# EUROPÄISCHE PATENTSCHRIFT

(11) **EP 2 496 539 B1**
(45) Veröffentlichungstag und Bekanntmachung des Hinweises auf die Patenterteilung: **03.09.2014**
(21) Anmeldenummer: 10779266.5
(22) Anmeldetag: 02.11.2010
(51) Int. Cl.: C07C 7/08, C07C 11/167, C10G 7/08, C10G 21/20, B01D 3/40

(54) **VERFAHREN ZUR AUFTRENNUNG EINES C4-SCHNITTES DURCH EXTRAKTIVDESTILLATION MIT EINEM SELEKTIVEN LÖSUNGSMITTEL**
METHOD FOR SEPARATING A C4 FRACTION BY MEANS OF EXTRACTIVE DISTILLATION USING A SELECTIVE SOLVENT
PROCÉDÉ DE SÉPARATION D'UNE COUPE C4 PAR DISTILLATION EXTRACTIVE AVEC UN SOLVANT SÉLECTIF

(30) Priorität: 03.11.2009 EP 09174947
(43) Veröffentlichungstag der Anmeldung: 12.09.2012
(73) Patentinhaber: BASF SE, 67056 Ludwigshafen (DE)
(72) Erfinder: HUGO, Randolf, 67246 Dirmstein (DE); PESCHEL, Werner, 67251 Freinsheim (DE); STABEL, Uwe, 67166 Otterstadt (DE); ZACHMANN, Harry, 67063 Ludwigshafen (DE); JÄGER, Michael, Laudenbach 69514 (DE)
(86) Internationale Anmeldenummer: PCT/EP2010/066595
(87) Internationale Veröffentlichungsnummer: WO 2011/054798

(56) Entgegenhaltungen:
- WO-A1-2008/006879

## Beschreibung

Die Erfindung betrifft ein Verfahren zur Auftrennung eines C₄-Schnittes durch Extraktivdestillation mit einem selektiven Lösungsmittel.

Der beispielsweise in Crackern anfallende so genannte C₄-Schnitt umfasst ein Gemisch von Kohlenwasserstoffen, wobei die C₄-Kohlenwasserstoffe, insbesondere 1-Buten, i-Buten sowie 1,3-Butadien überwiegen. Neben geringen Menge an C3- und C₅-Kohlenwasserstoffen enthält der C₄-Schnitt in der Regel C₃- und C₄-Acetylene, z.B. 1-Butin, Butein und Propin, insbesondere 1-Butin (Ethylacetylen) und Butenin (Vinylacetylen).

Die Gewinnung von 1,3-Butadien aus derartigen Gemischen ist wegen der geringen Unterschiede in den relativen Flüchtigkeiten ein kompliziertes destillationstechnisches Problem. Daher wird die Auftrennung durch eine so genannte Extraktivdestillation durchgeführt, das heißt eines Destillation unter Zugabe eines selektiven Lösungsmittels, das einen höheren Siedepunkt als das aufzutrennende Gemisch aufweist und das die Unterschiede in den relativen Flüchtigkeiten der aufzutrennenden Komponenten erhöht.

Durch Auswahl geeigneter selektiver Lösungsmittel ist es möglich, den C₄-Schnitt per Extraktivdestillation in einen Kopfstrom aufzutrennen, enthaltend die weniger als 1,3-Butadien löslichen Kohlenwasserstoffe, insbesondere Butane und Butene, sogenanntes Raffinat I, und ein mit den übrigen Kohlenwasserstoffen aus dem C₄-Schnitt, insbesondere 1,3-Butadien, den Butinen sowie gegebenenfalls 1,2-Butadien beladenes Lösungsmittel, woraus die genannten Kohlenwasserstoffe anschließend unter Rückgewinnung des Lösungsmittels ausgegast werden.

Die Kapazität bestehender Anlagen zur Extraktivdestillation ist insbesondere durch die folgenden Parameter festgelegt:
- die Temperatur des selektiven Lösungsmittels bei der Zuführung in die Extraktivdestillationskolonne,
- die Temperatur am Kopf der Kolonne aus der Raffinat I abgezogen wird,
- der Mengenstrom und die Temperatur des Rücklaufstromes an Raffinat I,
- die Anzahl der theoretischen Trennstufen in der Extraktivdestillationskolonne,
- die Lage der Zulaufstelle des C₄-Schnittes,
- die Dampf- und/oder Flüssigkeitsbelastung der Extraktivdestillationskolonne,
- der Druck in der Extraktivdestillationskolonne sowie
- die Lösungsmittel-Umlaufmenge.

Diese Parameter können nur begrenzt im Sinne einer Steigerung der Kapazität der Anlage verändert werden, weil technische oder wirtschaftliche Grenzen erreicht werden:

So ist die Temperatur, auf die das selektive Lösungsmittel vor der Zuführung desselben abgekühlt werden kann und die Kondensationstemperatur des Kopfstromes für Raffinat I durch das verfügbare Kühlmittel, in der Regel Flusswasser, begrenzt. Die Anzahl der theoretischen Trennstufen für eine gegebene Extraktivdestillationskolonne ist bei bestehenden Anlagen vorgegeben und bei Neuanlagen aus insbesondere wirtschaftlichen Gründen begrenzt. Schließlich kann die Dampf- und/oder Flüssigkeitsbelastung einer Kolonne nicht über einen bestimmten Bereich erhöht werden, ohne den ordnungsgemäßen Betrieb derselben nachteilig zu beeinflussen.

In den technisch verfügbaren Verfahren, welche zur Gewinnung von Butadien-1,3 aus C₄-Schnitten unterschiedliche Lösungsmittel einsetzen, sind jeweils große Lösungsmittelumlaufströme erforderlich. Pumpen, Kolonnen, Wärmeübertrager, Rohrleitungen, etc. sind daher entsprechend groß zu dimensionieren, was zu hohen Investitionskosten für Neuanlagen führt. Gleichzeitig sind damit für vorhandene Anlagen Grenzen für Kapazitätserhöhungen vorgegeben, da bei vergrößertem Lösungsmittelumlauf sehr viele Apparate und Maschinen geändert oder ersetzt werden müssen. Eine Kapazitätserhöhung unter Beibehaltung des Lösungsmittelumlaufstromes wäre aus wirtschaftlicher Sicht bedeutend attraktiver.

In EP-A 2 043 977 wurde daher vorgeschlagen, durch Abzug eines Energiestromes (Zwischenkühlung) im Bereich zwischen der Zulaufstelle des selektiven Lösungsmittels und dem Sumpfstrom, enthaltend Roh-Butadien, die Kapazität vorhandener Anlagen zu erhöhen. Ein flüssiger oder dampfförmiger Strom wird in außenliegenden Wärmeübertragern oder innenliegenden Wärmeübertragungseinheiten abgekühlt. Das Verfahren wird bevorzugt in zwei hintereinander geschalteten Kolonnen durchgeführt, wobei die Verbindungsleitungen zwischen den beiden Kolonnen für flüssiges bzw. dampfförmiges Medium die bevorzugten Stellen zum Abzug des Energiestromes sind. Hierbei wird der C₄-Schnitt in den Sumpf der ersten Kolonne zugefahren, d.h. oberhalb der bevorzugten Stelle für den Energieabzug.

Der gemäß dem Verfahren der EP-A 2 043 977 abzuführende Energiestrom ist sehr groß. Der Zugewinn an C₄-Kapazität liegt bei einer Abkühlung des flüssigen Sumpfstromes der ersten Kolonne um 10 K in der gleichen Größenordnung, wie er durch eine Abkühlung des am Kopf der ersten Kolonne zuzuführenden Stromes des selektiven Lösungsmittels um ca. 2,5 K wäre. Dabei ist der Sumpfstrom lediglich um die eingelösten C₄-Komponenten größer als der am Kopf der ersten Kolonne aufgegebene Lösungsmittelstrom. Der Wirkungsgrad der in EP 2 043 977 vorgeschlagenen Maßnahme ist somit in der bevorzugten Ausführungsform, wonach in der Anlage zur Extraktivdestillation eine erste Kolonne, in deren unteren Bereich der aufzutrennende C₄-Schnitt und in deren oberen Bereich das selektive Lösungsmittel aufgegeben wird, über eine Flüssigkeits- und eine Brückenleitung mit einer zweiten Kolonne verbunden ist, und Energie aus der Flüssigkeits- und/oder der Dampfleitung abgezogen wird, eher gering.

Es war daher Aufgabe der Erfindung, ein Verfahren zur Verfügung zu stellen, wonach die Kapazität bestehender Anlagen zur Auftrennung von C₄-Schnitten durch Extraktivdestillation mit einem selektiven Lösungsmittel mit vergleichbarem Aufwand deutlich stärker gesteigert werden kann. Insbesondere sollte der aufzutrennende Feedstrom stärker erhöht werden.

Die Aufgabe wird gelöst durch ein Verfahren zur Auftrennung eines C₄-Schnittes durch Extraktivdestillation mit einem selektiven Lösungsmittel durch Gegenstromführung des C₄-Schnittes und des selektiven Lösungsmittels in flüssiger Phase in einer Destillationseinheit (K I, K II) unter Abtrennung eines Kopfstromes, enthaltend die Butane und die Butene aus dem C₄-Schnitt sowie eines Sumpfstromes, enthaltend das selektive Lösungsmittel und die übrigen Komponenten des C₄-Schnittes, außer den Butanen und den Butenen, aus dem in weiteren Verfahrensschritten die übrigen Komponenten des C₄-Schnittes, außer den Butanen und den Butenen ausgegast werden, das dadurch gekennzeichnet ist, dass zwischen der Abzugsstelle für den Kopfstrom und der Zulaufstelle für den C₄-Schnitt in die Destillationseinheit (K I, K II) Energie aus der Destillationseinheit (K I, K II) abgezogen wird.

Bevorzugt wird die Stelle, von der Energie abgezogen wird, in der Destillationseinheit so positioniert, dass zwischen derselben und der Zulaufstelle für den C₄-Schnitt 15 % bis 70 %, bevorzugt 30 % bis 50 %, der Gesamtzahl der oberhalb der Zulaufstelle für den C₄-Schnitt vorhandenen theoretischen Trennstufen angeordnet sind.

Die Destillationseinheit umfasst eine oder mehrere, bevorzugt zwei Kolonnen.

Diese sind mit trennwirksamen Einbauten, die insbesondere als Betten von Füllkörperschüttungen, strukturierte Packungen oder Böden ausgebildet sind, ausgestattet.

Bei Kolonnen, die mit Betten aus Füllkörperschüttungen als trennwirksamen Einbauten ausgestattet sind, wird die Stelle, von der Energie abgezogen wird, sowie auch die Stelle, an der der abgekühlte Strom zurückgeführt wird, bevorzugt im Bereich zwischen zwei Betten angeordnet.

Bei einer Kolonne mit beispielsweise sechs Betten wird die Stelle, von der Energie abgezogen wird, bevorzugt oberhalb des zweiten, des dritten oder des vierten Bettes (bei Zählung der Betten von unten nach oben) angeordnet, insbesondere oberhalb des zweiten oder dritten Bettes positioniert.

Bei einer Kolonne, die mit fünf Betten ausgestattet ist, wird die Stelle, von der Energie abgezogen wird, bevorzugt oberhalb des ersten, des zweiten oder des dritten Bettes, bei Zählung von unten nach oben, angeordnet, insbesondere oberhalb des zweiten Bettes positioniert.

Um Energie aus der Destillationseinheit abzuziehen, wird von einer wie oben definierten Abzugsstelle der an der entsprechenden Stelle in der Destillationseinheit vorhandene Flüssigkeits- oder Gasstrom, bevorzugt der Flüssigkeitsstrom, abgezogen.

Bevorzugt kann hierbei der Gesamtstrom der an der entsprechenden Stelle vorhandenen Flüssigkeit, d.h. ein mit Komponenten aus dem C₄-Schnitt beladener Strom des selektiven Lösungsmittels, abgezogen werden, oder auch nur ein Teilstrom des mit Komponenten des C₄-Schnitts beladenen Stroms des selektiven Lösungsmittels, insbesondere etwa 80 bis 90 % des Gesamtstromes.

Zur praktischen Ausführung hierzu kann insbesondere in der entsprechenden Kolonne ein Kaminboden eingebaut werden, von dem die Flüssigkeit abgezogen, mit einer Pumpe einem Wärmetauscher zugeführt und erneut unterhalb des Kaminbodens der Kolonne wieder zugefahren wird.

Sofern nicht der Gesamtstrom, sondern nur ein Teilstrom der Flüssigkeit abgezogen wird, läuft der nicht abgezogene Teilstrom über auf den darunter befindlichen Verteiler oder Boden.

Indem lediglich ein Teilstrom des gesamten, mit Komponenten des C₄-Schnitts beladenen Stromes des selektiven Lösungsmittels abgezogen wird, können Kosten gespart werden, wobei sich gegebenenfalls ein Behälter mit Standregelung einsparen lässt.

Der aus der Kolonne abgezogene Strom, insbesondere Flüssigkeitsstrom, wird zur Temperaturabsenkung über einen Wärmetauscher geleitet. Dieser kann bevorzugt mit Wasser, aber auch mit Luft gekühlt werden.

Je weiter oben der Abzug zur Kühleinrichtung angeordnet wird, desto größer wird bei gleichbleibendem Wärmeabzugsstrom der Zugewinn an Verarbeitungskapazität durch Extraktivdestillation der C₄-Schnitten. Bei gegebener Kühlwassertemperatur oder - im Falle eines Luftkühlers - der Umgebungstemperatur sind der Abkühlung des beladenen Stromes des selektiven Lösungsmittels Grenzen gesetzt. Da die Temperatur in der Destillationseinheit oben am niedrigsten ist und nach unten hin ansteigt, kann mehr Wärme abgezogen werden, je weiter unten der abzukühlende Strom abgezogen wird. Zudem kann es bei Abkühlung des beladenen Lösungsmittelstromes zu einem Phasenzerfall in zwei flüssige Phasen kommen (Lösungsmittelphase und Phase, enthaltend Komponenten des C₄-Schnittes, worunter die Trennleistung leiden würde. Dieses Problem tritt umso weniger auf, je tiefer der abzukühlende Strom abgezogen wird.

Die optimale Stelle für den Abzug des Stromes, über den Energie aus der Destillationseinheit entnommen wird, kann der Fachmann aufgrund bekannter verfahrenstechnischer Überlegungen mit Hilfe des Simulationsprogramms Aspen ® Plus der Fa. Aspen Technology Inc. ermitteln.

Im Wesentlichen sind die Kapazitäten der Wärmeübertrager sowie die vorhandenen Kolonneneinbauten entsprechend der gegebenen hydraulischen Belastung anzupassen.

Es wurde gefunden, dass es möglich ist, die Kapazität von Anlagen zur Extraktivdestillation von C₄-Schnitt in einfacher Weise mit geringem apparativen und energetischen Aufwand durch Beeinflussung des Temperaturprofils zu verbessern, indem Energie aus dem Verfahren abgezogen wird, ohne dass hierfür Endwerte des Temperaturverlaufs verändert werden.

Beispielsweise kann durch Abziehen einer entsprechenden Menge an thermischer Energie eine Temperaturabsenkung von bis zu 10 °C oder auch von bis zu 15 °C erreicht werden.

Die Erfindung ist nicht eingeschränkt bezüglich der konkreten Durchführung des Verfahrens zur Auftrennung eines C₄-Schnitts durch Extraktivdestillation mit einem selektiven Lösungsmittel. Insbesondere kann es sich auch um ein Verfahren handeln, bei dem zusätzlich zur Extraktivdestillation eine Selektivhydrierung acetylenischer Verunreinigungen durchgeführt wird.

Das erfindungsgemäße Verfahren ist beispielsweise anwendbar auf eine Verfahrensvariante, bei der die Destillationseinheit, in der die Extraktivdestillation durchgeführt wird, aus zwei Kolonnen gebildet ist, die untereinander mit einer Flüssigkeitsleitung und einer Dampfleitung verbunden sind. In dieser Verfahrensvariante wird vorteilhaft Energie aus der Flüssigkeits- und/oder der Dampfleitung, bevorzugt aus der Flüssigkeitsleitung, abgezogen. In der Verfahrensvariante mit zwei Kolonnen wird der ersten Kolonne bevorzugt dem unteren Bereich derselben der C₄-Schnitt, bevorzugt dampfförmig zugeführt, und im Gegenstrom hierzu, im oberen Bereich der ersten Kolonne, das selektive Lösungsmittel. Am Kopf der ersten Kolonne wird ein Strom, enthaltend die leichter als 1,3-Butadien siedenden Kohlenwasserstoffe, insbesondere Butane und Butene, so genanntes Raffinat I, abgezogen.

In einer weiteren bevorzugten Ausführungsform umfasst die Destillationseinheit eine erste Kolonne und eine zweite Kolonne, die untereinander mit einer Flüssigkeitsleitung und einer Dampfleitung verbunden sind, wobei Flüssigkeit aus der ersten Kolonne über die Flüssigkeitsleitung in die zweite Kolonne und Dampf aus der zweiten Kolonne über die Dampfleitung in die erste Kolonne geleitet wird, wobei die Zulaufstelle für den C₄-Schnitt im oberen Bereich der zweiten Kolonne angeordnet ist, oberhalb der Zulaufstelle für den C₄-Schnitt mindestens eine theoretische Trennstufe vorgesehen ist und Energie aus der Flüssigkeitsleitung und/oder der Dampfleitung abgezogen wird.

Die erste Kolonne ist über eine Flüssigkeits- und eine Dampfleitung mit einer zweiten Kolonne verbunden, in der die Abtrennung eines mit den übrigen Komponenten aus dem C₄-Schnitt gegenüber den Butanen und Butenen beladenen selektiven Lösungsmittels über Sumpf erfolgt. Hieraus werden in einem oder mehreren weiteren Apparaten die weiteren Komponenten des C₄-Schnitts aus dem selektiven Lösungsmittel abgetrennt, wobei insbesondere ein Roh-1,3-Butadienstrom erhalten wird.

Als Roh-1,3-Butadien wird in bekannter Weise ein Kohlenwasserstoffgemisch verstanden, das das Wertprodukt 1,3-Butadien in einem Anteil von mindestens 80 Gew.-%, bevorzugt mindestens 90 Gew.-%, oder auch mindestens 95 Gew.-%, Rest Verunreinigungen, enthält.

Zusätzlich ist es möglich, an einer oder mehreren weiteren Stellen der Destillationseinheit Flüssigkeit und/oder Gas abzuziehen, über einen außen liegenden Wärmetauscher zu führen und darin abzukühlen und erneut in die Destillationseinheit an derselben Stelle zurückzuführen, an der der Abzug der Flüssigkeit oder des Gases erfolgt war, oder an einer hiervon verschiedenen Stelle.

Zusätzlich oder alternativ ist es möglich, Flüssigkeit und/oder Gas über innenliegende Kühleinrichtungen in der Destillationseinheit zu kühlen.

Vorteilhaft wird der C₄-Schnitt der Destillationseinheit teilweise oder vollständig in flüssiger Form zugeführt.

Der vorliegend als Ausgangsgemisch einzusetzende sogenannte C₄-Schnitt ist ein Gemisch von Kohlenwasserstoffen mit überwiegend vier Kohlenstoffatomen pro Molekül. C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, Butene, 1,3-Butadien, daneben geringe Mengen an C₃- und C₅-Kohlenwasserstoffen, sowie Butine, insbesondere 1-Butin (Ethylacetylen) und Butenin (Vinylacetylen). Dabei beträgt der 1,3-Butadiengehalt im Allgemeinen 10 bis 80 Gew.-%, vorzugsweise 20 bis 70 Gew.-%, insbesondere 30 bis 60 Gew.-%, während der Gehalt an Vinylacetylen und Ethylacetylen im Allgemeinen 5 Gew.-% nicht übersteigt.

Geeignete selektive Lösungsmittel für das erfindungsgemäße Verfahren sind zum Beispiel Butyrolacton, Nitrile wie Acetonitril (ACN), Propionitril, Methoxypropionitril, Ketone wie Aceton, Furfurol, N-alkylsubstituierte niedere aliphatische Säureamide, wie Dimethylformamid (DMF), Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im Allgemeinen werden N-alkylsubstituierte niedere aliphatische Säureamide oder N-alkylsubstituierte cyclische Säureamide verwendet. Besonders vorteilhaft sind Dimethylformamid und insbesondere N-Methylpyrrolidon.

Es können jedoch auch Mischungen dieser Lösungsmittel untereinander, zum Beispiel von N-Methylpyrrolidon mit Acetonitril, sowie Mischungen dieser Lösungsmittel mit Colösungsmitteln, wie Wasser, Ethern, zum Beispiel Methyl-tert.-butylether, Ethyl-tert.-butylether, Propyl-tert.-butylether, n- oder iso-Butyl-tert.-butylether und/oder Alkoholen, zum Beispiel Isoproanol, eingesetzt werden.

Besonders geeignet ist N-Methylpyrrolidon, bevorzugt in wässriger Lösung, insbesondere mit 7 bis 10 Gew.-% Wasser, besonders bevorzugt mit 8,3 Gew.-% Wasser.

Besonders geeignet sind auch DMF, ACN, sowie Mischungen von ACN mit Wasser und/oder Alkohol.

Die Erfindung wird im Folgenden anhand einer Zeichnung und eines Ausführungsbeispiels näher erläutert.

Es zeigen im Einzelnen:
- Figur 1: die schematische Darstellung einer Destillationseinheit mit zwei Kolonnen K I und K II, an die sich eine Reinigungs- und Entgasungseinheit K III anschließt, ohne Abzug von Energie (Stand der Technik), und
- Figur 2: die schematische Darstellung einer Destillationseinheit mit zwei Kolonnen K I und K II, wobei die Kolonne K I gegenüber der Verschaltung in Abbildung 1 verkürzt und die Kolonne K II verlängert ist, mit einer bevorzugten Stelle für den Abzug von Energie (erfindungsgemäß).

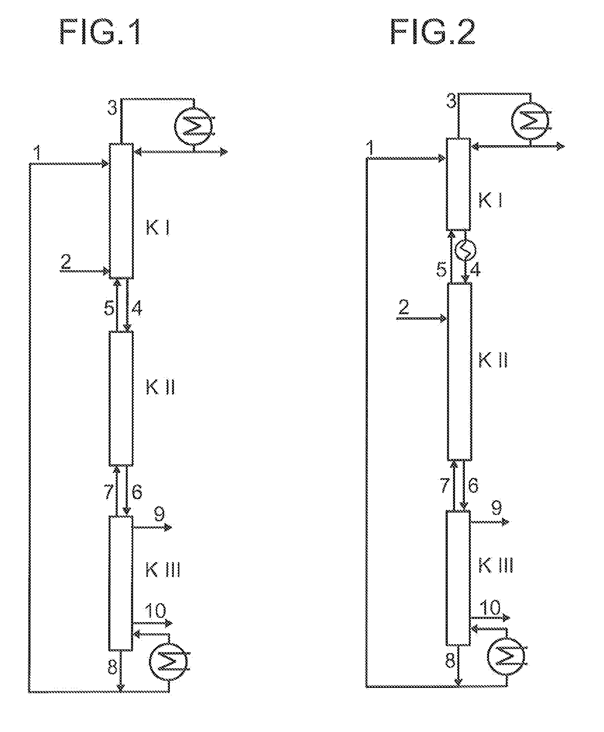

Figur 1 zeigt eine Destillationseinheit, umfassend zwei Destillationskolonnen K I und K II, der ein selektives Lösungsmittel, Strom 1, im oberen Bereich der Kolonne K I, und ein aufzutrennender C₄-Schnitt, Strom 2, im unteren Bereich der Kolonne K I zugeführt wird. Aus der Kolonne K I wird ein Kopfstrom 3 abgezogen, in einem außen liegenden Wärmeübertrager kondensiert, teilweise aus dem Verfahren abgezogen und im übrigen als Rücklauf auf der Kolonne K I aufgegeben.

Die Kolonne K I ist über eine Flüssigkeitsleitung 4 und eine Dampfleitung 5 mit der Kolonne KII verbunden. Aus der Kolonne KII wird ein Sumpfstrom 6 abgezogen, enthaltend selektives Lösungsmittel, das mit den restlichen Komponenten des C₄-Schnitts außer den Butanen und Butenen beladen ist. Dieser Strom wird einer Reinigungs- und Entgasungseinheit K III zugeführt und hieraus ein Roh-1,3-Butadien enthaltender Strom 9 sowie ein C₄-Acetylene sowie Hochsieder, das heißt Verbindungen mit einem Siedepunkt höher als dem Siedepunkt von 1,3-Butadien, enthaltender Strom 10, abgezogen, unter Verbleib von gereinigtem selektivem Lösungsmittel, das als Sumpfstrom 8 abgezogen wird.

Die Reinigungs- und Entgasungseinheit K III ist über eine Dampfleitung 7 mit der Kolonne K II verbunden.

In der in Figur 2 dargestellten bevorzugten Ausführungsvariante wird aus der Verbindungsleitung 4 (Flüssigkeitsleitung) Energie abgezogen, über einen Wärmetauscher gekühlt und die gekühlte Flüssigkeit der Kolonne K II zugeführt.

Die nachfolgenden Beispiele ergeben sich auf rechnerischer Basis mit Hilfe des Simulationsprogramms Aspen ® Plus der Fa. Aspen Technology Inc.

### Ausführungsbeispiel 1 (Vergleich), keine Zwischenkühlung

In einem Verfahren zur Auftrennung eines C₄-Schnitts durch Extraktivdestillation mit 8,3 Gew.-% Wasser enthaltendem N-Methylpyrrolidon als selektivem Lösungsmittel in einer Anlage entsprechend der schematischen Darstellung in Figur 1 wird ein C₄-Strom am Sumpf der Kolonne K I gasförmig aufgegeben. Das Verhältnis des NMP-Stromes 1 zum Kopf der Kolonne K I, bezogen auf den Strom 2 (C₄-Schnitt), beträgt 9,77:1, d.h. bei einer Verfügbarkeit von 300 t/h selektivem Lösungsmittel zum Kopf der Kolonne K I können 30,7 t/h des C₄-Schnittes verarbeitet werden.

### Ausführungsbeispiel 2 (Vergleich) mit Zwischenkühlung im Sumpf der Kolonne K I, unmittelbar unterhalb des C₄-Zulaufs (Strom 2)

In einem Verfahren zur Auftrennung eines C₄-Schnitts durch Extraktivdestillation mit 8,3 Gew.-% Wasser enthaltendem N-Methylpyrrolidon als selektivem Lösungsmittel in einer Anlage entsprechend der schematischen Darstellung in Figur 1 wird der Flüssigkeitsstrom 4 aus der Kolonne K I abgezogen, um 10 K abgekühlt und in das Verfahren an der Abzugsstelle desselben recycliert. Dadurch kann das Verhältnis des NMP-Stromes 1 zum Kopf der Kolonne K I, bezogen auf den C₄-Schnitt (Strom 2), bei sonst gleichen Randbedingungen auf 9,39:1 reduziert werden, d.h. mit 300 t/h selektivem Lösungsmittel zum Kopf der Kolonne K I können 31,9 t/h C₄-Schnitt verarbeitet werden, was einer Kapazitätssteigerung gegenüber dem Ausführungsbeispiel 1 um 3,9 % entspricht.

### Ausführungsbeispiel 3 (erfindungsgemäß) mit Zwischenkühlung oberhalb des zweiten von insgesamt sechs Betten in der Kolonne K I

In einem Verfahren zur Auftrennung eines C₄-Schnitts durch Extraktivdestillation mit 8,3 Gew.-% Wasser enthaltendem N-Methylpyrrolidon als selektivem Lösungsmittel in einer Anlage entsprechend der schematischen Darstellung in Figur 1 wird im unteren Teil der Kolonne K I ein Flüssigkeitsstrom abgezogen, in einem Wärmeübertrager abgekühlt und unmittelbar unterhalb der Abzugsstelle der Kolonne K I wieder zugeführt. Die Abzugsstelle ist so gewählt, dass von den in K I insgesamt vorhandenen Trennstufen 2/3 oberhalb und 1/3 unterhalb der Abzugsstelle sind. Es wird die gesamte Flüssigkeitsmenge, die an dieser Stelle anfällt, abgezogen, wobei der im Wärmeübertrager abgeführte Wärmestrom exakt gleich ist, wie der Wärmestrom in Ausführungsbeispiel 2. Die Temperaturerniedrigung beträgt 10,4 K. Das Verhältnis des Lösungsmittelsstromes 1 zum Kopf der Kolonne K I, bezogen auf den Strom 2, bei ansonsten gleichen Randbedingungen beträgt in diesem Fall 8,71:1, d.h. mit 300 t/h selektivem Lösungsmittel zum Kopf der Kolonne K I können 34,4 t/h C₄-Schnitt verarbeitet werden, was gegenüber Ausführungsbeispiel 1 ohne Zwischenkühlung (zum Vergleich) einer Kapazitätssteigerung um 12,1 % entspricht. Gegenüber der Variante mit Zwischenkühlung unmittelbar unterhalb der Zulaufstelle des Stromes 2 zur Kolonne K I gemäß Ausführungsbeispiel 2 beträgt die Kapazitätssteigerung 7,9 %.

### Ausführungsbeispiel 4 (erfindungsgemäß) mit Zwischenkühlung oberhalb des dritten von insgesamt sechs Betten in der Kolonne K I

In einem Verfahren zur Auftrennung eines C₄-Schnitts durch Extraktivdestillation mit 8,3 Gew.-% Wasser enthaltendem N-Methylpyrrolidon als selektivem Lösungsmittel in einer Anlage entsprechend der schematischen Darstellung in Figur 1 wird in der Mitte der Kolonne K I ein Flüssigkeitsstrom abgezogen, in einem Wärmeübertrager abgekühlt und unmittelbar unterhalb der Abzugsstelle der Kolonne K I wieder zugeführt. Die Abzugsstelle ist so gewählt, dass von den in K I insgesamt vorhandenen Trennstufen die Hälfte oberhalb und die andere Hälfte unterhalb der Abzugsstelle sind. Es wird die gesamte Flüssigkeitsmenge, die an dieser Stelle anfällt, abgezogen, wobei der im Wärmeübertrager abgeführte Wärmestrom exakt gleich ist, wie der Wärmestrom in Ausführungsbeispiel 2. Das Verhältnis des NMP-Stromes 1 zum Kopf der Kolonne K I, bezogen auf den Strom 2, bei ansonsten gleichen Randbedingungen beträgt in diesem Fall 8,55:1, d.h. mit 300 t/h selektivem Lösungsmittel zum Kopf der Kolonne K I können 35,1 t/h C₄-Schnitt verarbeitet werden.

Gegenüber Ausführungsbeispiel 1 ohne Zwischenkühlung (zum Vergleich) ergibt sich eine Kapazitätssteigerung um 14,3 %. Gegenüber der Variante mit Zwischenkühlung unmittelbar unterhalb der Zulaufstelle des aufzutrennenden C₄-Schnittes, Strom 2, zur Kolonne K I gemäß Ausführungsbeispiel 2 beträgt die Kapazitätssteigerung 10,0 %.

### Ausführungsbeispiel 5 (erfindungsgemäß) mit Zwischenkühlung oberhalb des vierten von insgesamt sechs Betten in der Kolonne K I

In einem Verfahren zur Auftrennung eines C₄Schnitts durch Extraktivdestillation mit 8,3 Gew.-% Wasser enthaltendem N-Methylpyrrolidon als selektivem Lösungsmittel in einer Anlage entsprechend der schematischen Darstellung in Figur 1 wird in der Mitte der Kolonne K I ein Flüssigkeitsstrom abgezogen, in einem Wärmeübertrager abgekühlt und unmittelbar unterhalb der Abzugsstelle der Kolonne K I wieder zugeführt. Die Abzugsstelle ist so gewählt, dass von den in K I insgesamt vorhandenen Trennstufen 1/3 oberhalb und 2/3 unterhalb der Abzugsstelle sind. Es wird die gesamte Flüssigkeitsmenge, die an dieser Stelle anfällt, abgezogen, wobei der im Wärmeübertrager abgeführte Wärmestrom exakt gleich ist, wie der Wärmestrom in Ausführungsbeispiel 2. Das Verhältnis des NMP-Stromes 1 zum Kopf der Kolonne KI, bezogen auf den Strom 2, bei ansonsten gleichen Randbedingungen beträgt in diesem Fall 8,43:1, d.h. mit 300 t/h selektivem Lösungsmittel zum Kopf der Kolonne K I können 35,6 t/h C₄-Schnitt verarbeitet werden.

Gegenüber Ausführungsbeispiel 1 ohne Zwischenkühlung (zum Vergleich) ergibt sich eine Kapazitätssteigerung um 16,0 %. Gegenüber der Variante mit Zwischenkühlung unmittelbar unterhalb der Zulaufstelle des aufzutrennenden C₄-Schnittes, Strom 2, zur Kolonne KI gemäß Ausführungsbeispiel 2 beträgt die Kapazitätssteigerung 11,6 %.

## Patentansprüche

1. Verfahren zur Auftrennung eines C₄-Schnittes (2) durch Extraktivdestillation mit einem selektiven Lösungsmittel (1) durch Gegenstromführung des C4-Schnittes und des selektiven Lösungsmittels (1) in flüssiger Phase in einer Destillationseinheit (KI, KII) unter Abtrennung eines Kopfstromes (3), enthaltend die Butane und die Butene aus dem C₄-Schnitt sowie eines Sumpfstromes (6), enthaltend das selektive Lösungsmittel und die übrigen Komponenten des C₄-Schnittes, außer den Butanen und den Butenen, aus dem in weiteren Verfahrensschritten die übrigen Komponenten des C4-Schnittes, außer den Butanen und den Butenen ausgegast werden, **dadurch gekennzeichnet, dass**
zwischen der Abzugsstelle für den Kopfstrom (3) und der Zulaufstelle für den C₄-Schnitt (2) in die Destillationseinheit (K I, K II) Energie aus der Destillationseinheit (KI, K II) abgezogen wird.

2. Verfahren nach Anspruch 1, **dadurch gekennzeichnet, dass** die Stelle, von der Energie abgezogen wird, in der Destillationseinheit (K I, K II) so positioniert ist, dass zwischen derselben und der Zulaufstelle für den C₄-Schnitt (2) 15 bis 70 % der Gesamtzahl der oberhalb der Zulaufstelle für den C₄-Schnitt (2) vorhandenen theoretischen Trennstufen angeordnet sind.

3. Verfahren nach Anspruch 1, **dadurch gekennzeichnet, dass** die Stelle, von der Energie abgezogen wird, in der Destillationseinheit (K I, K II) so positioniert ist, dass zwischen derselben und der Zulaufstelle für den C₄-Schnitt (2) 30 bis 50 % der Gesamtzahl der oberhalb der Zulaufstelle für den C₄-Schnitt (2) vorhandenen theoretischen Trennstufen angeordnet sind.

4. Verfahren nach einem der Ansprüche 1 bis 3, **dadurch gekennzeichnet, dass** Energiemenge abgezogen wird, die einer Temperaturabsenkung von bis zu 15°C entspricht.

5. Verfahren nach einem der Ansprüche 1 bis 4, **dadurch gekennzeichnet, dass** die Destillationseinheit eine erste Kolonne (K I) und eine zweite Kolonne (K II) umfasst, die untereinander mit einer Flüssigkeitsleitung (4) und einer Dampfleitung (5) verbunden sind, wobei Flüssigkeit aus der ersten Kolonne (K I) über die Flüssigkeitsleitung (4) in die zweite Kolonne (K II) und Dampf aus der zweiten Kolonne (K II) über die Dampfleitung (5) in die erste Kolonne (K I) geleitet wird, dass die Zulaufstelle für den C₄-Schnitt (2) im oberen Bereich der zweiten Kolonne (K II) angeordnet ist, wobei oberhalb der Zulaufstelle für den C₄-Schnitt (2) mindestens eine theoretische Trennstufe vorgesehen ist, und dass Energie aus der Flüssigkeitsleitung (4) und/oder der Dampfleitung (5) abgezogen wird.

6. Verfahren nach einem der Ansprüche 1 bis 5, **dadurch gekennzeichnet, dass** an einer oder mehreren Stellen aus der Destillationseinheit (KI, KII) Flüssigkeit und/oder Gas abgezogen, in einem außenliegenden Kühler abgekühlt und in die Destillationseinheit (K I, K II) an derselben Stelle oder an einer von der Stelle des Abzugs der Flüssigkeit und/oder des Gases verschiedenen Stelle zurückgeführt wird.

7. Verfahren nach einem der Ansprüche 1 bis 6, **dadurch gekennzeichnet, dass** Flüssigkeit und/oder Dampf über einen in der Destillationseinheit (K I, K II) innenliegenden Kühler geleitet wird.

8. Verfahren nach einem der Ansprüche 1 bis 7, **dadurch gekennzeichnet, dass** als selektives Lösungsmittel N-Methylpyrrolidon, bevorzugt in wässriger Lösung, insbesondere mit 7 bis 10 Gew.-% Wasser, besonders bevorzugt mit 8,3 Gew.-% Wasser, eingesetzt wird.

9. Verfahren nach einem der Ansprüche 1 bis 7, **dadurch gekennzeichnet, dass** als selektives Lösungsmittel Dimethylformamid eingesetzt wird.

10. Verfahren nach einem der Ansprüche 1 bis 7, **dadurch gekennzeichnet, dass** als selektives Lösungsmittel Acetonitril oder eine Mischung hiervon mit Wasser und/oder einem oder mehreren Alkoholen eingesetzt wird.

## Claims

1. A process for fractionating a C₄ fraction (2) by extractive distillation using a selective solvent (1) by conveying the C₄ fraction and the selective solvent (1) in countercurrent in the liquid phase in a distillation unit (K I, K II) and separating off an overhead stream (3) comprising the butanes and the butenes from the C₄ fraction and also a bottom stream (6) comprising the selective solvent and the other components of the C₄ fraction apart from the butanes and the butenes from which, in further process steps, the other components of the C₄ fraction apart from the butanes and the butenes are stripped as gas, wherein energy is taken off from the distillation unit (K I, K II) between the point at which the overhead stream (3) is taken off and the point at which the C₄ fraction (2) is fed into the distillation unit (K I, K II).

2. The process according to claim 1, wherein the point from which energy is taken off is preferably positioned in the distillation unit (K I, K II) so that from 15% to 70% of the total number of theoretical plates present above the point at which the C₄ fraction (2) is fed in are arranged between the point from which energy is taken off and the point at which the C₄ fraction (2) is fed in.

3. The process according to claim 1, wherein the point from which energy is taken off is preferably positioned in the distillation unit (K I, K II) so that from 30% to 50% of the total number of theoretical plates present above the point at which the C₄ fraction (2) is fed in are arranged between the point from which energy is taken off and the point at which the C₄ fraction (2) is fed in.

4. The process according to any of claims 1 to 3, wherein the quantity of energy taken off corresponds to a temperature reduction of up to 15°C.

5. The process according to any of claims 1 to 4, wherein the distillation unit comprises a first column (K I) and a second column (K II) which are connected with one another by a liquid line (4) and a vapor line (5), with liquid from the first column (K I) being conveyed via the liquid line (4) into the second column (K II) and vapor from the second column (K II) being conveyed via the vapor line (5) into the first column (K I) and the point at which the C₄ fraction (2) is fed in being arranged in the upper region of the second column (K II), at least one theoretical plate being provided above the point at which the C₄ fraction (2) is fed in and energy being taken off from the liquid line (4) and/or the vapor line (5).

6. The process according to any of claims 1 to 5, wherein liquid and/or gas is taken off from the distillation unit (K I, K II) at one or more points, cooled in an external cooler and fed back into the distillation unit (K I, K II) at the same point or at a point different from the point at which the liquid and/or gas was taken off.

7. The process according to any of claims 1 to 6, wherein liquid and/or vapor are/is passed through an internal cooler in the distillation unit (K I, K II).

8. The process according to any of claims 1 to 7, wherein N-methylpyrrolidone, preferably in aqueous solution, in particular with from 7 to 10% by weight of water, particularly preferably with 8.3% by weight of water, is used as selective solvent.

9. The process according to any of claims 1 to 7, wherein dimethylformamide is used as selective solvent.

10. The process according to any of claims 1 to 7, wherein acetonitrile or a mixture thereof with water and/or one or more alcohols is used as selective solvent.

## Revendications

1. Procédé pour la séparation d'une fraction en C₄ (2) par distillation extractive avec un solvant sélectif (1) par conduite à contre-courant de la fraction en C₄ et du solvant sélectif (1) en phase liquide dans une unité de distillation (KI, KII) avec séparation d'un courant de tête (3), contenant les butanes et les butènes provenant de la fraction en C₄, ainsi que d'un courant de pied (6), contenant le solvant sélectif et les composants restants de la fraction en C₄, hormis les butanes et les butènes, à partir duquel dans d'autres étapes du procédé les autres composants de la fraction en C₄, hormis les butanes et les butènes, sont évacués sous forme de gaz, **caractérisé**
**en ce qu'**entre le point d'extraction du courant de tête (3) et le point d'alimentation de la fraction en C₄ (2) dans l'unité de distillation (K I, K II) de l'énergie est extraite de l'unité de distillation (K I, K II).

2. Procédé selon la revendication 1, **caractérisé en ce que** le point duquel l'énergie est extraite est positionné dans l'unité de distillation (K I, K II) de telle sorte qu'entre celui-ci et le point d'alimentation de la fraction en C₄ (2) soient disposés 15 à 70 % du nombre total d'étages de séparation théoriques présents au-dessus du point d'alimentation de la fraction en C₄ (2).

3. Procédé selon la revendication 1, **caractérisé en ce que** le point duquel l'énergie est extraite est positionné dans l'unité de distillation (K I, K II) de telle sorte qu'entre celui-ci et le point d'alimentation de la fraction en C₄ (2) soient disposés 30 à 50 % du nombre total d'étages de séparation théoriques présents au-dessus du point d'alimentation de la fraction en C₄ (2).

4. Procédé selon l'une quelconque des revendications 1 à 3, **caractérisé en ce qu'**est évacuée une quantité d'énergie qui correspond à un abaissement de la température de jusqu'à 15°C.

5. Procédé selon l'une quelconque des revendications 1 à 4, **caractérisé en ce que** l'unité de distillation comprend une première colonne (K I) et une deuxième colonne (K II) qui sont reliées l'une à l'autre par un conduit pour liquides (4) et un conduit pour vapeurs (5), du liquide étant conduit hors de la première colonne (K I) par le biais du conduit pour liquides (4) dans la deuxième colonne (K II) et de la vapeur étant conduite hors de la deuxième colonne (K II) par le biais du conduit pour vapeurs (5) dans la première colonne (K I), **en ce que** le point d'alimentation de la fraction en C₄ (2) est disposé dans la région supérieure de la deuxième colonne (K II), au moins un étage de séparation théorique étant prévu au-dessus du point d'alimentation de la fraction en C₄ (2), et **en ce que** de l'énergie est évacuée du conduit pour liquides (4) et/ou du conduit pour vapeurs (5).

6. Procédé selon l'une quelconque des revendications 1 à 5, **caractérisé en ce qu'**un liquide et/ou un gaz est/sont extrait(s) en un ou plusieurs points de l'unité de distillation (K I, K II), refroidi(s) dans un refroidisseur externe et renvoyé(s) dans l'unité de distillation (K I, K II) en le même point ou en un point différent du point de l'évacuation du liquide et/ou du gaz.

7. Procédé selon l'une quelconque des revendications 1 à 6, **caractérisé en ce qu'**on fait passer le liquide et/ou la vapeur sur un refroidisseur se trouvant dans l'unité de distillation (K I, K II).

8. Procédé selon l'une quelconque des revendications 1 à 7, **caractérisé en ce qu'**on utilise comme solvant sélectif la N-méthylpyrrolidone, de préférence en solution aqueuse, en particulier avec 7 à 10 % en poids d'eau, de façon particulièrement préférée avec 8,3 % en poids d'eau.

9. Procédé selon l'une quelconque des revendications 1 à 7, **caractérisé en ce qu'**on utilise comme solvant sélectif le diméthylformamide.

10. Procédé selon l'une quelconque des revendications 1 à 7, **caractérisé en ce qu'**on utilise comme solvant sélectif l'acétonitrile ou un mélange de celui-ci avec l'eau et/ou un ou plusieurs alcools.
